# EUROPEAN PATENT SPECIFICATION

(11) **EP 1 726 263 B1**
(45) Date of publication and mention of the grant of the patent: **29.04.2009**
(21) Application number: 06010475.9
(22) Date of filing: 22.05.2006
(51) Int. Cl.: A61B 17/02

(54) **Surgical retractor blade support**
Chirurgische Retraktorblatthalterung
Support d'écarteur chirurgical

(30) Priority: 23.05.2005 ES 200501172 U
(43) Date of publication of application: 29.11.2006
(73) Proprietor: ANSABERE SURGICAL S.L., 31110 Noain (Navarra) (ES)
(72) Inventor: Insausti Isturiz, Juan José, 08940 Cornellà de Llobregat Barcelona (ES)
(74) Representative: Isern-Jara, Nuria

(56) References cited:
- WO-A-20/05040953
- US-A- 4 813 401
- US-A- 4 930 932
- US-A- 5 297 538
- US-A- 5 512 038
- US-A1- 2002 026 101
- US-A1- 2004 193 018

## Description

### OBJECT OF THE INVENTION

The object of this invention is a surgical retractor blade support that incorporates significant innovations and advantages compared to the current support arrangements used for the same purpose.

More specifically the new invention support is an arrangement that is basically simple, easy to handle and quick to assemble and dismantle, and which makes it easy to handle by the surgeon.

### BACKGROUND TO THE INVENTION

At the present time, in the technical field of surgery the use of retractor blades as separation elements at the edges of the surgical incision is well known. The assembling of said retractor blades is carried out, in general, around bars making up a frame suitable for making the actions to be carried out easier during an operation or around base elements similar to the stated frame.

In particular, the assembly elements or those related to the retractor blades around the frame or similar items are clamps with complex arrangements that make the work more difficult and slow down both the work of the surgeon and equally the assembly and dismantling operation of the assembly.

A retractor blade support of the kind mentioned above is disclosed in US 2004/193018 A1 whose document disclose a surgical retractor system comprises a support frame and an instrument mounting assembly. Also, it is known by applicant several retractor blade supports with similar features in comparison to present invention, for instance those disclosed in WO 2005/040953, US2002/026101 and US 4 930 932. However, in none of applications above mentioned it has been located a return mechanism to be simple and effective as described in the independent claim 1.

### DESCRIPTION OF THE INVENTION

The surgical retractor blade support object of this invention solves the previously stated disadvantages, moreover, providing other advantages that will be clear from the description which is attached below.

In order to do this and to be more specific, the surgical retractor blade support of the invention is as defined in claim 1.

In particular, the groove making up the construction of the retractor blade support section has an opening at its inner end and is connected to the inner hollow section, in such a way that the central axis will be in direct contact with the retractor blade to be fixed.

Likewise, the retractor blade support section is provided with a means of retaining the retractor blade. The stated retaining means is made from a peg and at least one opening through which the said peg passes in at least one of the arms forming the fork.

The central axis for coupling the holding element comprises a return mechanism that has a shaft and an elastic spring, in such a way on inserting the retractor blade in the groove making up the fork this mechanism will be retracted exercising pressure in the opposite direction on the retractor blade.

Thanks to these characteristics an improved support is obtained for surgical retractor blades with an arrangement that is simple, easy to handle and quick to assemble and dismantle, and which makes it easy to handle by the surgeon.

In order to complete the description that will be made below and for the purpose of providing a better understanding of its characteristics, a set of drawings is attached to this present description in which the figures being by way of illustration and are not by way of limitation on the invention, in which the most significant details of the invention are shown:

### BRIEF DESCRIPTION OF THE DRAWINGS

Figure 1 shows a perspective view of the surgical retractor blade support of the invention;
Figure 2 shows a perspective view of the support shown in the previous figure without the holding element;
Figure 3 shows a perspective view of the holding element;
Figure 4 shows an elevation and section view of the holding element shown in the previous figure; and
Figure 5 shows a plan view of the holding element shown in the two previous figures.

### DESCRIPTION OF A PREFERRED EMBODIMENT

In view of the figures commented on, and in accordance with the numbering adopted, a preferred embodiment of the invention can be seen in same that is not by way of limitation on the invention, which consists of a surgical retractor blade support.

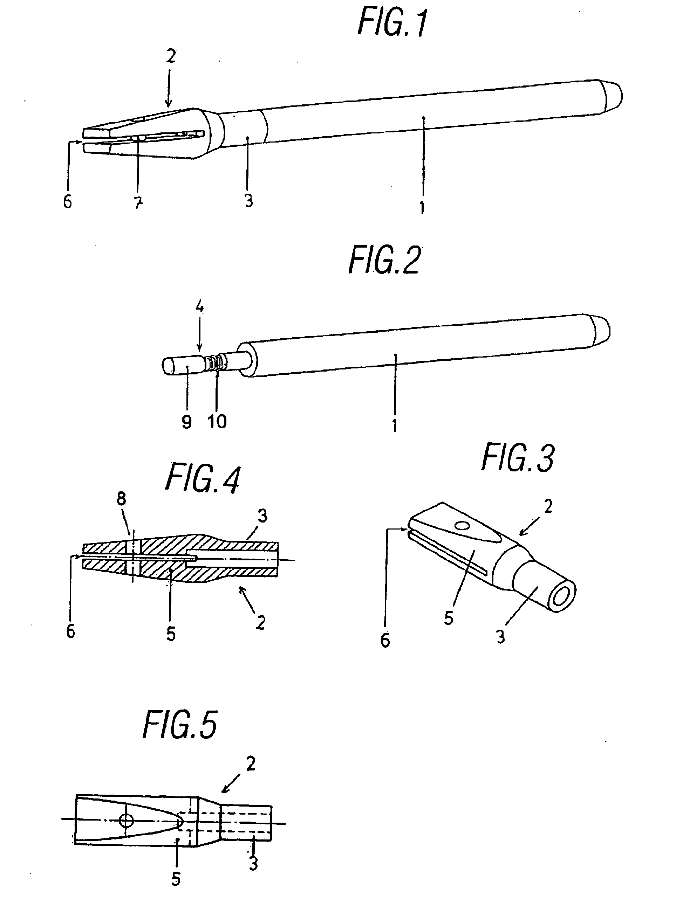

As and how can be seen in figures 1, 2, 3, 4 and 5, the retractor blade support is of the type that has a connection area (1), by way of a bar, for the connection of the support to the base element that is appropriate for the operation to be carried out, and holding means for the fixing and stabilisation of the valve, wherein said holding means has a holding element (2) made from a hollow section (3) for the coupling to an axis (4) that protrudes from one of the ends of the bar making up the connection area (1), and a retractor blade support section (5) with a fork shaped arrangement.

The groove (6) making up the construction of the retractor blade support fork has an opening at its inner end and is connected to the hollow section (3) in such a way that the central axis (4) will be in direct contact with the retractor blade to be fixed.

Likewise, the fork is provided with a means of retaining the retractor blade. The stated retaining means is made from a peg (7) and at least one opening (8) through which the said peg (7) passes in at least one of the arms forming the fork.

The central axis (4) for coupling the holding element (2) comprises a return mechanism that has a shaft (9) and an elastic spring (10), in such a way on inserting the retractor blade in the groove (6) making up the fork this mechanism will be retracted exercising pressure in the opposite direction on the retractor blade (not shown).

The details, shapes, sizes and other accessorial elements, likewise the materials used for the construction of the support of the invention can suitably be substituted for others that do not deviate from the scope defined by the claims which are included below.

## Claims

1. A surgical retractor blade support having a connection area (1), by way of a bar, for connecting the support to a base element, and holding means for fixing and stabilising a retractor blade, said holding means having a holding element (2) with a hollow section (3) for coupling to an axis (4) that protrudes from one of the ends of the bar making up the connection area (1), and a retractor blade support section (5) with a fork shaped arrangement, wherein the axis (4) for coupling the holding element (2) comprises a return mechanism, **characterised in that** the return mechanism has a shaft (9) and an elastic spring (10).

2. A surgical retractor blade support according to claim 1, wherein the retractor blade support section (5) has a groove (6) having an opening at its inner end and is connected to the hollow section (3) in such a way that the axis (4) will be in direct contact with the retractor blade to be fixed.

3. A surgical retractor blade support according to claim 1, wherein the retractor blade support section (5) is provided with a means of retaining the retractor blade, and said retaining means is fitted in an arm of the retractor blade support section (5).

4. A surgical retractor blade support, according to claim 3, **characterised in that** the retractor blade retaining means comprises a peg (7) and at least one opening (8) for the insertion of said peg (7).

## Patentansprüche

1. Eine Halterung für chirurgische Klappen, die über einen Verbindungsbereich (1) mit einem Stab für die Verbindung der Halterung mit einer Unterlage verfügt, sowie über Befestigungsvorrichtungen zur Befestigung und Stabilisierung einer Klappe, wobei die genannten Befestigungsvorrichtungen über ein Befestigungselement (2) mit einem hohlen Abschnitt (3) verfügen, für die Verbindung mit einer Achse (4), die aus einem Ende des Stabs hervorsteht, der den Verbindungsbereich bildet (1), und einen gabelförmigen Abschnitt zur Befestigung der Klappe (5), in dem die Achse (4) zum Aufstecken des Befestigungselements (2) über eine Rückstellvorrichtung verfügt, die sich **dadurch** auszeichnet, dass die Rückstellvorrichtung über eine Achse (9) und eine elastische Feder verfügt (10).

2. Eine Halterung für chirurgische Klappen (1) nach Anspruch 1, in der der Abschnitt zur Befestigung der Klappe (5) über eine Nut verfügt (6), die am inneren Ende eine Öffnung hat und mit dem hohlen Abschnitt (3) verbunden ist, so dass die Achse (4) in direktem Kontakt zu der zu befestigenden Klappe steht.

3. Eine Halterung für chirurgische Klappen (1) nach Anspruch 1, in welche der Abschnitt zur Befestigung der Klappe (5) über Haltevorrichtungen für die Klappe verfügt und sich diese Haltevorrichtungen auf einem Arm des Abschnitts zur Befestigung der Klappe befinden (5).

4. Eine Halterung für chirurgische Klappen (1) nach Anspruch 3, die **dadurch gekennzeichnet ist, dass** die Haltevorrichtungen für die Klappe einen Stift (7) sowie mindestens eine Öffnung (8) zum Einstecken des genannten Stifts (7) umfassen.

## Revendications

1. Un support pour valves chirurgicales qui possède une zone de rapport (1), au moyen d'une barre, pour unir le support et un élément de base, et des moyens de fixation pour tenir et stabiliser une valve, ces moyens de fixation ayant un élément de fixation (2) muni d'un tronçon creux (3) permettant l'accouplement à un axe (4) qui dépasse de l'une des extrémités de la barre qui constitue la zone de rapport (1), et un tronçon de fixation de la valve (5) en forme de fourche, dans lequel l'axe (4) pour accoupler l'élément de fixation (2) comprend un mécanisme de retour, **caractérisé par le fait que** le mécanisme de retour comprend un axe (9) et un ressort élastique (10).

2. Un support pour valves chirurgicales (1) selon la revendication 1, dans lequel le tronçon de fixation de la valve (5) possède une rainure (6) qui présente une ouverture à son extrémité intérieure et est reliée au tronçon creux (3) de sorte que l'axe (4) sera en contact direct avec la valve à fixer.

3. Un support pour valves chirurgicales (1) selon la revendication 1, dans lequel le tronçon de fixation de la valve (5) est muni de moyens de retenue de la valve, et ces moyens de retenue sont disposés sur un bras du tronçon de fixation de la valve (5).

4. Un support pour valves chirurgicales (1) selon la revendication 3, **caractérisé par le fait que** les moyens de retenue de la valve comprennent une clavette (7) et au moins un orifice (8) pour l'insertion de ladite clavette (7).
